# EUROPEAN PATENT APPLICATION

(11) **EP 3 868 417 A1**
(43) Date of publication of application: **25.08.2021**
(21) Application number: 20157823.4
(22) Date of filing: 18.02.2020
(51) Int. Cl.: A61M 1/16, A61M 1/28

(54) **WEARABLE AND PORTABLE DEVICE FOR RECIRCULATING FLOW DIALYSIS**

(71) Applicant: ICinnovation BV, 5688 RX Oirschot (NL)
(72) Inventor: Simonis, Frank, 5688 RX Oirschot (NL)
(74) Representative: Verhees, Godefridus Josephus Maria

(57) **Abstract**

The invention relates to a dialysis device that provides recirculating flow dialysis in a wearable and portable format. It uses an exchangeable purification unit with sorbents for the in-situ regeneration of dialysate. The invented dialysis device comprises a carrier that is mounted on a replaceable cartridge. The carrier holds the electronics, user-interface, actuators and sensors. It actuates, controls and monitors the dialysis operation. The cartridge is a replaceable part that is connected to the patient via a flexible tubing. It consists of a reusable housing with a memory chip and holds a disposable inlay containing the purification unit with sorbents, fluid lines, connectors and a nanofilter. The cartridge is intended for use during the day, as a wearable system. The cartridge can be enlarged with an extension set to offer more capacity. The extended cartridge is intended to be used during the night as a portable bedside device.

## Description

### FIELD OF THE INVENTION

The invention relates to a dialysis device in a wearable and / or portable format for home use.

### BACKGROUND OF THE INVENTION

Acute renal failure and end stage kidney disease can be treated with different dialysis modalities, depending on patient characteristics and hospital resources. Peritoneal dialysis (PD) can be first choice in situations like hypotension, heart failure, disturbed coagulation, or difficult venous access. Existing peritoneal dialysis techniques are CAPD (continuous ambulatory peritoneal dialysis) and APD (automated peritoneal dialysis). Both techniques use a peritoneal dialysis fluid (typically 2L) that is entered in the abdomen and that is drained after saturation with toxins. This is performed 4 to 5 times per day (manually, CAPD) or at night (automated, APD). The main disadvantage of PD is the limited efficacy. This is due to the intermittent procedure of CAPD and APD and the rapid saturation of the dialysate in the abdomen. A second drawback is the high glucose concentration in the dialysate that is added as osmotic agent in order to extract excess fluid from the patient. A high peak in glucose concentration induces a reaction of the peritoneal membrane resulting in a lowering of its functionality over time hereby limiting the technique survival (typically 5 years). Continuous flow peritoneal dialysis (CFPD) where fresh dialysate is circulated continuously is a much more effective treatment than conventional PD. The continuous circulation enhances the efficacy of the peritoneal dialysis treatment and high peaks in glucose concentration can be prevented. So far, continuous flow peritoneal dialysis requires connection - and hereby immobilization - to a stationary dialysis machine for long periods per day. This is not practical and hinders its application.

Several efforts have been undertaken to develop a wearable and/or portable device that would allow for continuous flow peritoneal dialysis in a more practical setting. Best known system is described in patent application US2011/0184340 where a system is presented that recirculates peritoneal dialysate which is being regenerated by a sorbent cartridge. The sorbent system is described in patent application US2011/0171713. It contains four components: active carbon (binding organic toxins such as creatinine, uric acid etc), immobilized urease (enzyme converting urea into ammonium and carbon dioxide), zirconium phosphate (binding cations such as ammonium, potassium, calcium, magnesium) and hydrous zirconium oxide (removing anions such as phosphate). This sorbent system has proven to be effective in toxin removal but commercialization is hampered due to some physiological drawbacks and high costs of the system. Main causes are: (1) the urease enzyme has a limited shelf life (2) the enzyme converts urea into large amounts of (toxic) ammonium that can only be removed by a large volume of zirconium phosphate (3) next to ammonium a large amount of carbon dioxide is formed that needs to be degased from the dialysate (4) the zirconium phosphate also binds magnesium ions, calcium ions and too much potassium ions that need to be re-infused separately (5) upon ion exchange, both the zirconium phosphate and zirconium oxide release sodium ions which is unfavourable for the electrolyte balance of the patient. The sodium release, limited shelf-life, need for degasing and re-infusing of electrolytes and the difficult control of ammonium are obstructing a successful market introduction. Similar systems are presented in others patent applications such as US2010/0314314 and US2014/8777892 but with the same drawbacks.

In patent application US2015/0290384 a different sorbent system is presented comprising active carbon (binding most of the organic toxins), a metal (Cu)-complexed chitosan (binding urea) and an uncomplexed chitosan (binding metal ions). In this system the unwanted release of metal (Cu) ions is a too high risk factor.

Patent application WO2016/190794 describes a sorbent system with a large amount of active carbon (organic toxins and urea), a phosphate resin (polystyrene complexed with Lanthanum or Ferric ions) and a cation exchange resin for the removal of potassium. Because of the poor absorption capacity for urea, this system demands for a large volume of carbon that counteracts a wearable system.

### SUMMARY OF THE INVENTION

It is an object of the present invention to overcome the above mentioned problems with unwanted release of ions and with a high volume of required sorbents (especially for urea) that counteracts wearability and leads to high costs.

The problem is solved according to the invention by a dialysis device that recirculates dialysate in a continuous or tidal mode and that makes use of a purification unit to regenerate the dialysate in-situ. The purification unit is held in a small cartridge for daytime use (wearable, carry-on system). The purification unit holds a volume of sorbents and - in case of tidal mode - a small dialysate volume. The sorbent system comprises two components: active carbon and a binder for anions. The sorbent system is designed to bind and remove toxins such as phosphate, small and middle molecules and protein bound toxins (creatinine, beta2-microglobulin, p-cresyl sulfate, indoxyl sulfate, hippuric acid, CMPF).

For dialysis during the night the cartridge is enlarged with an extension set (portable system). The function of the extension set is to remove also the bulk of the daily produced urea and potassium toxins. These are small ions that diffuse rapidly and can easily be removed in an overnight session of 8 hours. A second function is the release of an osmotic agent (such as glucose) needed for fluid extraction in case of peritoneal dialysis and -depending on the patient's need- release of base to neutralize the daily non-volatile acid production that is not sufficiently excreted by the kidneys in kidney failure.

The recirculation of the dialysate can be continuous or in tidal mode. Continuous circulation demands for a double lumen catheter or two separate catheters. As peritoneal patients usually have one single lumen catheter in place, it is expected that most peritoneal patients will use the system in tidal mode.

At a typical flow rate of 200ml/min the hourly recirculation of dialysate amounts to 6 liters when applying a tidal mode. For a nighttime session this means a dialysate recirculation volume of 48 liters. In current CAPD and APD procedures only 8-15 liters of dialysate are flushed in and out. The high recirculation volume augments the mass transfer in the peritoneal cavity. Hereby the efficacy of the dialysis treatment is enhanced. During daytime a similar volume can be recirculated at a reduced flow rate of 100ml/min.

The dialysis device can comprises a double patient line connected to a double lumen catheter or two separate catheters, or directly to the blood circuit via a regular blood access such as a shunt, fistula or a central venous catheter. Preferably, this dialysis device further comprises a dialyzer filter and two pump units that can be operated in dialysis mode.

### BRIEF DESCRIPTION OF THE DRAWINGS

The invention will be further elucidated below on the basis of drawings. These drawings show an embodiment of a prototype version according to the present invention. In the drawings:
Figure 1 shows a wearable device with daytime cartridge;Figure 2 shows two parts: (1) replaceable daytime cartridge with sorbents and fluidics (left, dark) and (2) carrier with electronics and sensors (right, white);
Figure 3 shows a device operating in tidal mode using a single patient line (and a single lumen catheter);
Figure 4 shows a portable device with extended nighttime cartridge;
Figure 5 shows a flow diagram daytime device in tidal mode using a single patient line (and single lumen catheter);
Figure 6 shows a flow diagram nighttime device in tidal mode, with extended cartridge (extension set), using a single patient line (and single lumen catheter);
Figure 7 shows an alternative flow diagram daytime device in tidal mode, with the nanofilter only in the dialysate flow entering the patient;
Figure 8 shows a flow diagram daytime device in continuous flow mode using two patient lines (and two catheters or double lumen catheter);
Figure 9 shows a flow diagram nighttime device with extension set in continuous flow mode using two patient lines (and two catheters or double lumen catheter);
Figure 10 shows a flow diagram daytime device in dialyzing mode, using a dialyzer filter and two pump units, using two patient lines (and two catheters or double lumen catheter);
Figure 11 shows a dynamic light scattering of sorbent purified dialysate filtered by a 0.2um filter and by a 3nm high flux dialyzer filter compared with ultrapure water;
Figure 12 shows the association between the mean dialysate flow rate (Qd, mL/min) and the peritoneal mass transfer area coefficient (MTAC, mL/min) of urea, creatinine and phosphate;
Figure 13 shows the situation when the device is used with two separate catheters;
Figure 14 shows a preferred single port double lumen catheter for use in tidal mode with one lumen delivering fluid at the top of the peritoneum and one lumen retracting fluid at the bottom; and
Figure 15 shows a double port double lumen catheter for use in continuous mode with one lumen delivering fluid at the top of the peritoneum and one lumen retracting fluid at the bottom.

### DETAILED DESCRIPTION OF THE INVENTION

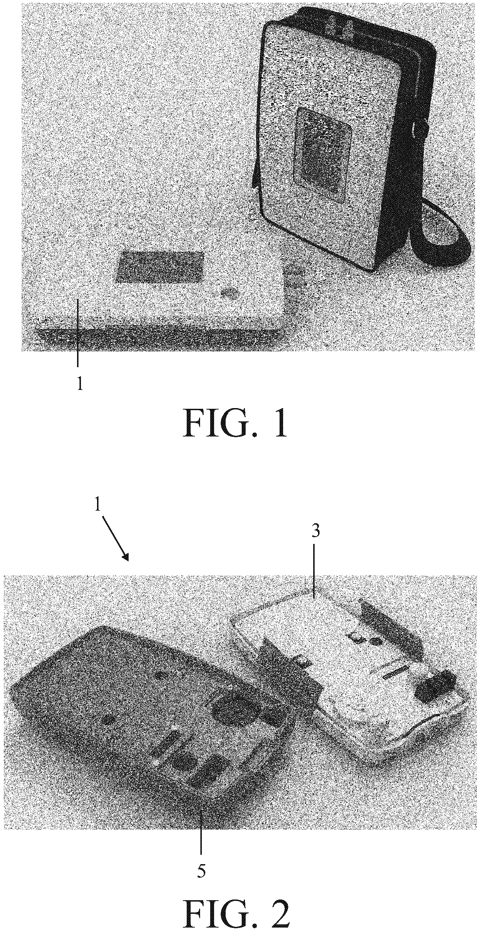

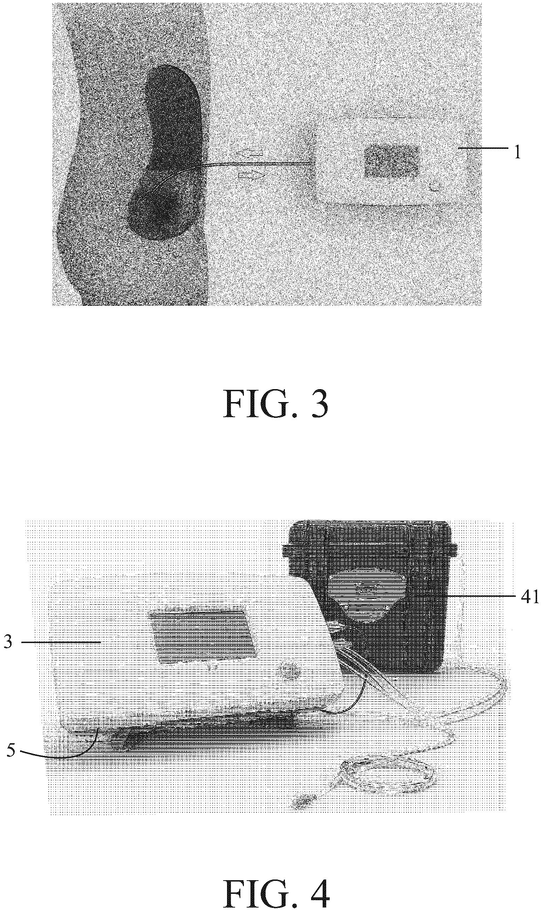

The problem is solved according to the invention by a dialysis device 1, see figure 1, that recirculates dialysate in a continuous or tidal mode and that makes use of a purification unit to regenerate the dialysate in-situ. The purification unit is held in a small cartridge 5, see figure 2, for daytime use (wearable, carry-on system), see figure 3.

The purification unit is designed to bind and remove toxins with a lower diffusivity such as phosphate, small and middle molecules and protein bound toxins (creatinine, beta2 microglobulin, p-cresyl sulfate, indoxyl sulfate, hippuric acid, CMPF).

For dialysis during the night the cartridge 5 is enlarged with an extension set 41, see figure 4 (extended cartridge, portable system). The function of the extension set 41 is to remove also the bulk of the daily produced urea and potassium toxins. These are small ions that diffuse rapidly and can easily be removed in an overnight session of 8 hours. A second function of the extension set is the release of an osmotic agent (such as glucose) needed for fluid extraction in case of peritoneal dialysis and - depending on the patient need - release of base to neutralize the daily non-volatile acid production that is not sufficiently excreted by the kidneys in kidney failure.

The recirculation of dialysate can be continuous or in tidal mode. Continuous circulation demands for a double lumen catheter or two separate catheters. As peritoneal patients usually have one single lumen catheter in place, it is expected that most peritoneal patients will use the system in tidal mode. This is achieved by a pump unit that reverses its direction at certain intervals.

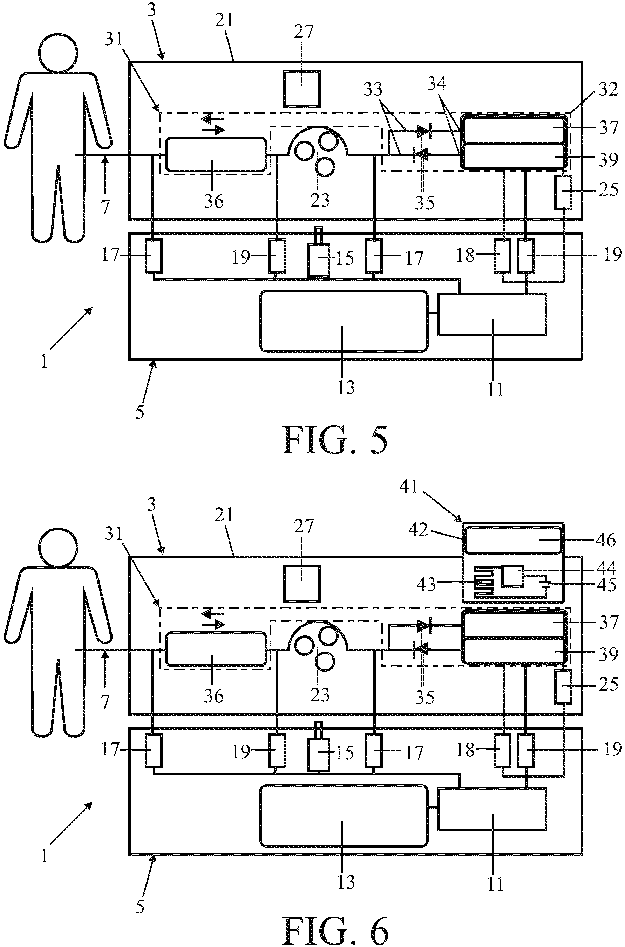

The invented dialysis device 1, see figure 5, comprises the following components:
i. a carrier 3 that holds the electronics 11, user-interface 13, actuators 15 and sensors 17, 18, 19. It actuates, controls and monitors the dialysis operation. It contains the following components:
   - housing with
      ∘ main button,
      ∘ alarm LED and buzzer,
      ∘ power connector (for battery and adapter),
      ∘ pump motor
   - electronics with
      ∘ sensors for temperature 18 (3x), pressure 19 (3x), air/bubble detectors 17 (2)
      ∘ display
      ∘ main control board (MAB) for the high level control (operation system), user interface and IT communication (webportal)
      ∘ hardware control board (HCB): operation of actuators and sensors, hardware safety control, data acquisition and data storage
ii. a cartridge 5, a replaceable part that is connected to the patient via a flexible tubing 7. The cartridge 5 consists of:
   - reusable housing 21 with
      ∘ pump 23
      ∘ leakage sensor 25
      ∘ memory chip 27 (CAB) for cartridge identification and data storage
   - purification unit/disposable inlay 31:
      ∘ compartment 32 with sorbents 37 and storage 39 for the tidal dialysate volume (tidal mode)
      ∘ tubings 33, connectors 34 and check valves 35 (tidal mode)
      ∘ particle filter 36

The cartridge 5 is intended for use during the day, as a wearable system (daytime cartridge, wearable daytime device) but can also be used overnight in specific situations.
iii. an extended cartridge, see figure 6, a cartridge 5 as described above but enlarged with an extension set 41. The extension set comprises the following components:
- case 42 with heating element 43, temperature controller 44 and power supply 45
- a dialysate reservoir 46 or as alternative options a compartment with sorbents for urea and potassium or an electrosorption unit

The extended cartridge is meant for nighttime use, as portable bedside device (nighttime cartridge, portable nighttime device) but can also be used during daytime when this is better suited.

Prototype versions of the device are shown in figure 1 (daytime device), figure 2 (carrier and cartridge), figure 3 (daytime device connected in tidal mode) and figure 4 (nighttime device with extension set).

Figure 1 shows a prototype version of the device 1. It consists of two parts: a carrier that is mounted on an interchangeable cartridge. These two parts are shown in figure 2. The carrier 3 contains the electronics with actuators, sensors and user-interface. This part actuates, controls and monitors the dialysis operation. The cartridge 5 is a replaceable part that holds the fluidic circuit and a purification unit for the regeneration of dialysate.

The device is connected to the peritoneal catheter of the patient via a fluidic line 7. For patients with a single lumen catheter the device operates in tidal model using a single fluid line. This is illustrated in figure 3. Continuous mode is also possible but requires a double lumen catheter or two separate catheters. Thanks to the small dimensions and weight (∼2kg) the patient can easily carry the device with him.

For use at nighttime the cartridge is expanded with an extension set. This so-called nighttime device is to be used as a portable bedside device and is shown in figure 4.

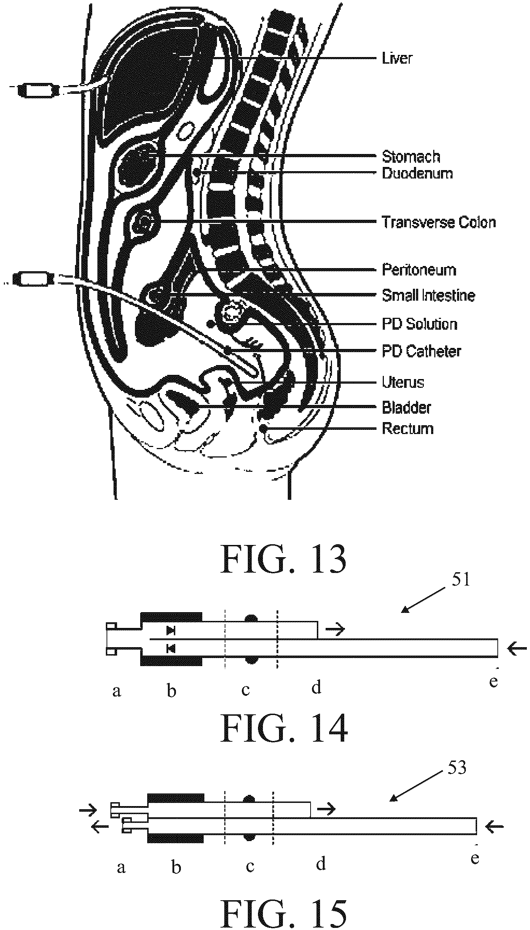

Patient connection: the device is connected to the peritoneal catheter of the patient via a single patient line to a single lumen or double lumen catheter (tidal mode) or via a double patient line to a double lumen catheter or two separate catheters (continuous mode). In the preferred embodiment the catheters are placed in such position that the delivery of fresh/refreshed dialysate occurs at the top of the peritoneal cavity and that the retraction of dialysate occurs at the bottom of the peritoneal cavity. This ensures an optimal contact between the dialysate and the peritoneal membrane and promotes a high mass transfer. The preferred positionings and layouts of the catheter(s) are depicted in figure 13 with two separate catheters that can be operated both in continuous and tidal mode, in figure 14 with a one port double lumen catheter that can only be operated in tidal mode and in figure 15 with a two port double lumen catheter that can be operated both in continuous and tidal mode. Most patients are equipped with a standard single lumen catheter. The enhancement in mass transfer by continuous or semi-continuous tidal flow with such single lumen catheter is limited because the in- and outgoing flow of such a catheter takes place in a limited volume of the peritoneal cavity. With two separate catheters or dedicated double lumen catheters as shown in figures 13-15, a larger volume and peritoneal membrane area can be accessed for mass transfer.

In fig. 13 two separate catheters are shown in the preferred position of one catheter delivering refreshed dialysate at the top of the peritoneum (liver, stomach) and the second catheter retracting fluid at the bottom of the peritoneal cavity. The catheters are placed in the preferred position of one catheter delivering refreshed dialysate at the top of the peritoneum (liver, stomach) and the second catheter retracting fluid at the bottom of the peritoneal cavity. This setup can be used both in continuous mode as well as in tidal mode.

The design of a single port double lumen catheter 51 with one lumen delivering fluid at the top of the peritoneum and one lumen retracting fluid at the bottom is shown in fig. 14. In this figure is a: luer lock connector, b: flow splitter with internal check valves, c: sub-skin cuff, d: outlet lumen delivering refreshed dialysate at the top of the peritoneum (liver, stomach) and e: entrance lumen retracting fluid from the peritoneal cavity at the bottom. The double lumen catheter has in-built check valves and a flow splitter.

The design of a double port double lumen catheter 53 with one lumen delivering fluid at the top of the peritoneum and one lumen retracting fluid at the bottom is shown in fig. 15. In this figure is a: two individual luer lock connectors, b: header, c: sub-skin cuff, d: outlet lumen delivering refreshed dialysate at the top of the peritoneum (liver, stomach) and e: entrance lumen retracting fluid from the peritoneal cavity at the bottom.

Fluid flow: dialysate is extracted and returned to the patient using a pump unit. In the preferred embodiment this is a peristaltic pump with a silicone or similar biocompatible tubing. The dialysate is regenerated by a purification unit containing sorbents and then filtered by a nanofilter before it is returned to the patient.

Figure 5 depicts the fluid circuit of the daytime device in tidal mode using one patient line (connected to one single lumen catheter). Peritoneal dialysate is withdrawn from the abdomen via a nanofilter by a peristaltic pump and stored in a reservoir. Here the dialysate is cleansed by sorbents that absorb the toxins. When the reservoir is full (tidal volume reached) the pump unit reverses and the cleansed dialysate is returned to the patient via the same nanofilter. The process is monitored by sensors for temperature, pressure, fluid leakage and air/gas-inclusions.

In figure 6 the fluid circuit is shown for the nighttime device, again in tidal mode, but now including the extension set. In the preferred embodiment the dialysate extracted from the patient is sent to the extension set and then to the sorbent/tidal reservoir before it is returned to the patient. But it also possible to send the extracted dialysate first to the sorbent/tidal reservoir and then to the extension set. In that case the two check valves in figure 6 should be inverted.

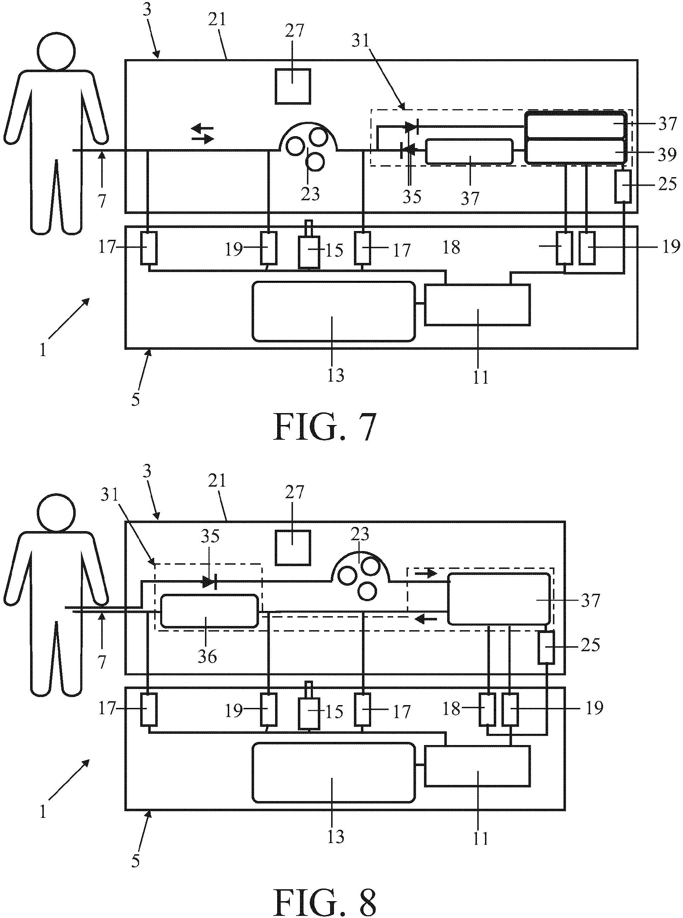

Fig. 5 presents the flow diagram of a daytime system in tidal mode using a single fluidic line connected to the patient's peritoneal catheter. The flow scheme for the nighttime system with the extension set to create an enlarged cartridge is depicted in fig 6, also for tidal mode. In fig 5 and 6 the nanofilter is positioned in the patient line. The dialysate flow is then filtered in both directions: the dialysate flow to the peritoneal cavity is filtered to remove potential particles and fines released from the sorbents whereas the flow extracted from the peritoneal cavity is filtered to block large molecules. Large proteins such as albumin and other large molecules such as high molecular weight glucose polymer (large molecular icodextrin, cyclo dextrin, highly branched cyclic dextrin, cluster dextrin) cannot pass the nanofilter and are not removed by the device. In general, removal of these large molecules is undesired because of enhanced protein catabolism in case of high dialysis related protein loss (albumin) and osmotic pressure (ultrafiltration). However, in case of patients with less of such needs, an alternative approach is recommended as depicted in fig 7. Figure 7 shows an alternative fluidic circuit where the nanofilter is used for filtering the return flow of cleansed dialysate only and not for filtering the extracted dialysate from the patient's abdomen. Here the nanofilter is placed directly downstream of the purification unit and used as a particle filter solely. In this case the performance of the device is not effected by the dead volume of the nanofilter.

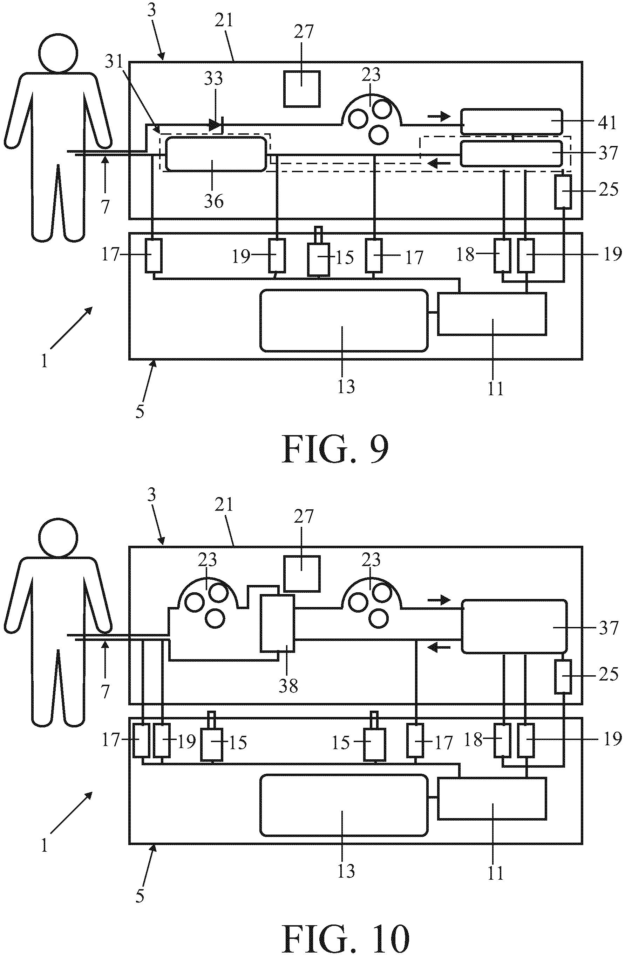

In continuous mode the device has two patient lines: one line to extract fluid and one line to return the refreshed dialysate. The related diagrams for continuous mode are given in fig 8 (daytime device) and 9 (nighttime device). Figure 8 represents the fluidic circuit of the daytime device in continuous mode using two separate fluidic lines for the withdrawal and return of dialysate (requires two catheters or one double lumen catheter). In continuous mode the internal reservoir only contains the sorbents. The fluidic circuit of the nighttime version is shown in figure 9. The patient lines are connected to two separate peritoneal catheters or one double lumen catheter. The systems in fig 8 and 9 make use of one peristaltic pump with the nanofilter placed directly downstream of the sorbent unit. In case it is preferred not to remove the larger molecules (albumin, high molecular weight glucose) from the peritoneal cavity a setup can be applied as depicted in fig 9 where a dialyzer is used to exchange toxins from the peritoneal cavity to the device. The fluidic content in the abdomen is then being dialyzed similar to the procedure of a hemodialysis machine. Such a setup requires two pumping units. The scheme of fig 10 is also applicable for hemodialysis. For hemodialysis the patient lines are connected to the hemodialysis blood access ports (shunt/fistula or CVC catheter).

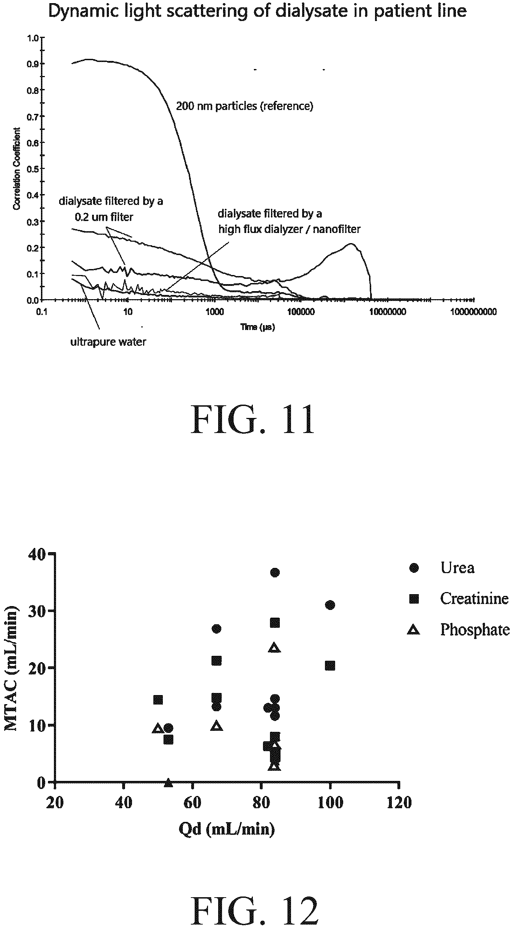

In the fluidic schemes of figure 8 and 9 the nanofilter 37 is placed in the dialysate return line. An alternative scheme is depicted in figure 10 where the peritoneal dialysate in the abdomen is being dialyzed via a dialyzer filter 38. This requires two pumping units 23. This setup is also applicable for hemodialysis. In case of hemodialysis the patient lines are connected to a regular blood access such as a shunt, fistula or central venous catheter. Blood is then withdrawn via fluidic line 7 and circulated through the dialyzer filter. Figure 11 shows the results of a dynamic light scattering (DLS) analysis of sorbent purified dialysate after filtration by a 0.2um filter and after filtration by a 3nm filter (high flux dialyzer). It is shown that submicron fines from the sorbents are not adequately blocked by a 0.2um filter and that a nanofilter is required to obtain an ultrapure quality.

Particle filter: the purified dialysate is filtered before it is returned to the patient. A standard 0.2um bacterial filter has proven to be insufficient in blocking submicron fines that can be released from the sorbents upon handling. This is demonstrated in fig 10. Therefore a nanofilter (nanofiltration membrane) is applied with a poresize of 1-10nm. In the preferred embodiment this nanofilter consists of a filter with a poresize of < 4nm, more in particular a dialyzer filter with a poresize in the range of 2-3nm. Such a dialyzer filter can be a low, middle or high flux dialyzer filter. The nanofilter may also be used to filter the dialysate flow that is withdrawn from the patient. Such a configuration is depicted in fig 5 and 6. Here the nanofilter blocks passage of large molecules (>10kD for a low flux filter, >30kDa for a high flux filter) and avoids their removal by the cartridge. Herewith loss of useful components such as albumin and high molecular weight glucose polymers are minimized. In such configurations the preferred embodiment will contain a high flux filter to allow removal of middle molecules such as beta2microglobulin. The combination of the nanofilter with a dialysate filling with high molecular weight glucose polymer (preferably >30kDa, maltodextrin, highly branched cyclic dextrin) as an osmotic agent would allow very low glucose additions for ultrafiltration as these molecules cannot pass the filter and are retained in the peritoneal cavity.

Purification unit: the disposable inlay of the cartridge holds a volume of sorbents and - in case of tidal mode- a small dialysate volume. The two volumes can be in two separate compartments or can be combined in one overall compartment. The design and in- and outlet of the compartment(s) is such that the risk for shortcut-flow is minimal. In the preferred embodiment the disposable inlay is made of a flexible bag in view of low manufacturing costs and ease of sterilization.

Sorbents: the sorbent system is designed to bind toxins with a low diffusivity and comprises of two components: (1) active carbon for the removal of organic toxins and (2) a phosphate binder such as ferric oxyhydroxide or lanthanum carbonate or carrier materials (polystryrene, starch) containing metal complexes of ferric or lanthanum. In the preferred embodiment the phosphate binder is a polystyrene modified with ferric oxyhydroxide. The sorbent volume may range from 100-2000ml, but typically will amount to 400-600ml. Next to binding of toxins the sorbents also act as a buffer system for glucose (active carbon) and base such as bicarbonate or lactate (phosphate binder), see below.

Preloading: sorbents are preloaded with specific compounds to achieve a neutral behavior (no adsorption or release of electrolytes) or a specific adsorption or release (bicarbonate, lactate, glucose). The sorbents are hereto equilibrated with a conditioning fluid of neutral pH (6-7.4) containing electrolytes (Na⁺, Mg²⁺, Ca²⁺, Cl-), bicarbonate, lactate and/or glucose ions. Bicarbonate and lactate ions are trapped by the phosphate binder. The trapped bicarbonate and lactate ions are released upon ion exchange with the phosphate from patient's dialysate during the treatment. The release of bicarbonate and lactate will contribute to a healthy acid base balance in the patient, namely neutralization of the daily non-volatile acid production that is not sufficiently excreted by the kidneys in kidney failure. The preloading concentrations are typically similar to the concentrations in the peritoneal dialysate of the extension set, but can also be lower or higher in case absorption or additional release is demanded. Preloading with magnesium and calcium ions is usually not needed and omission of these ions will eliminate the chance on possible precipitation of calcium and magnesium carbonate during storage. In case formation of glucose degradation products might occur during sterilization (e.g. during heat sterilization) it is recommended to omit glucose in the preloading of the sorbents. The active carbon will initially adsorb glucose from the dialysate solution but the adsorbed glucose will be released later on (partially) when the glucose level drops. The active carbon serves as a glucose buffer and provides peak shaving and stabilization of the glucose level. In very specific situations the sorbents might be preloaded also with other constituents such as vitamines and anti-oxidants such as ascorbic acid, N-acetylcysteine or glutathione to preserve the peritoneal membrane against oxidative stress.

Tidal mode: the tidal dialysate volume may range from 50-500ml but typically will amount to 200-300ml. This tidal volume is cycled in and out of the peritoneal cavity at a flow rate of 50-300ml/min. At a flow rate of typically 200ml/min in tidal mode the hourly recirculation of dialysate amounts to 6 liters. For a nighttime session this means a dialysate recirculation volume of 48 liters. In current CAPD and APD procedures only 8-15 liters of dialysate is flushed in and out. The high recirculation volume augments the mass transfer along the peritoneal membrane. Hereby the efficacy of the dialysis treatment is enhanced. During daytime a similar volume can be recirculated at a flow rate of 100ml/min.

Fluid management: in case of continuous circulation (two catheters/ double lumen catheter) the patient can select a suitable flow rate typically in the range of 50-300ml/min. The pump speed is set according this flow rate. In the case of tidal mode (single lumen catheter), the fluidic flow is defined by two adjustable parameters: the tidal volume that goes in and out of the peritoneal cavity and the flow rate. The tidal volume can be controlled e.g. by a timer during the cycle of entering dialysate into the abdomen. The cycle time is then equal to the tidal volume divided by the flow rate. At the end of this cycle, the pump is reversed in direction and dialysate is pumped out of the cavity into the tidal reservoir of the device. A sensor monitors the filling of the reservoir during this cycle. This can be any type of sensor. In case of the preferred embodiment where the tidal reservoir is made of a flexible bag the sensor can be a simple pressure or force sensor.

Air/bubble detection: the dialysate going in and out of the peritoneal cavity is monitored by an air/gas bubble detector, typically an ultrasonic detector that is clamped on the patient line at the outlet of the device. Detection of an air/gas bubble will raise an alarm and will lead to an automatic stop of the device. A second air/bubble detector is placed in-between the particle filter and the sorbent compartment. When air or a gas bubble is detected in tidal mode during the cycle that dialysate is entered into the peritoneal cavity, the pump direction is reversed and the air or gas bubble is pumped back into the sorbent compartment where it is trapped (internal air/bubble removal system).

Prevention of under/overfilling: to limit the risk of unintended under- or overfilling of the peritoneal cavity the system is equipped with sensors that monitor the filling pressure (pressure sensors), fluid loss (leakage sensor) and air inleak (air/bubble detectors).

Detection of leakage: leakage might occur due to a bad connection or a mechanical failure in the tubing or fluidic system. Fluidic loss is detected by a leakage sensor, e.g. a conductivity sensor. Air inleak is detected by one of the air/bubble detectors.

Detection blockage fluid line: blockage of the fluid line is detected by a pressure/force sensor in or on the patient line.

Extension set: the cartridge extension set 41 depicted in Figure 6 comprises of a case 42 with a heating element 43, a temperature control unit 44 and a power supply 45. It may hold a dialysate reservoir 46 of 5-15L, typically 7-10L. In the preferred embodiment this reservoir 46 has a volume of 9L and is made of flexible bag that can be placed in the case 42. This reservoir 46 is filled with a traditional peritoneal dialysate formulation containing NaCl, MgCl₂, CaCl₂, Na-bicarbonate and/or Na-lactate and glucose and/or icodextrin. The actual composition is fine-tuned by a medical doctor according to the patient needs. The reservoir is recirculated by the device as depicted in figures 6 and 9. The function of the reservoir is to add storage capacity for toxins such as urea and for potassium and to release the glucose needed for ultrafiltration (fluid extraction) and base -if necessary-for neutralization of the daily non-volatile acid production that is not sufficiently excreted. The active carbon in the cartridge acts as a buffer for the glucose: it adsorbs glucose at the start when the glucose concentration is relatively high and releases the glucose when the glucose concentration drops due to dilution (ultrafiltration) and reabsorption by the patient. The glucose level is therefore stabilized and the maximum peak concentration can be kept at a much lower level than in traditional PD.

Alternative extension set: the extension set may hold as alternative option to the dialysate reservoir an electrocatalytic sorption unit as described in WO2012060700 and WO2015060716. The content of these two patent application is herewith incorporated by reference. When the electrocatalytic sorption unit is equipped with a potassium binder (cation exchanger) it provides the capacity for removing both urea and potassium. The volume of such an extension set is typically 1-1.5L. This alternative option requires a separate infusion or release system for an osmotic agent (glucose, icodextrin or similar) and for an anti-oxidant (ascorbic acid, N-acetylcysteine, glutathione).

Alternative extension set: the extension set may hold as alternative option to the dialysate reservoir a compartment with sorbents for urea and potassium. This may reduce the volume of the extension set to 2-3L. This option requires a separate infusion or release system for glucose or similar osmotic agent.

### EXPERIMENTAL RESULTS

### Example 1: In vitro performance

Device prototypes as depicted in figures 1-6 were connected to a dialysate bags of 2L volume each, holding dialysate effluent collected from peritoneal dialysis patients (overnight Extraneal filling). The prototypes were equipped with cartridges holding 325ml active carbon, 135ml phosphate binder (ferricoxyhydroxide) and were operated in tidal mode with a tidal volume of 300ml. The experiments were carried out with daytime cartridges and also with nighttime cartridges (extended cartridges). The extension set comprised of a 10L dialysate reservoir filled with Physioneal 35 (Baxter). The dialysate bags were placed on a heating plate at 37C. The prototypes recirculated the effluent in tidal mode for 8 hours at a flow rate of 100 ml/min (daytime cartridge) or 200ml/min (nighttime cartridge). Every hour the effluent was spiked with urea, creatinine, phosphate and potassium to simulate transport of the solutes form the intravascular space into the peritoneal cavity of a patient. The cumulative removal of toxins by the cartridges is listed in table 1

**Table 1. In-vitro experiment with 2L PD effluent treated for 8 hrs: toxin removal by a daytime cartridge (460ml sorbents) and by a nighttime cartridge with additional 10L dialysate reservoir extension.**

| Removal (mmol) | Daytime cartridge | Nighttime cartridge |
|---|---|---|
| | - 325ml carbon | - 325ml carbon |
| | - 135ml phosphate binder | - 135ml phosphate binder |
| | | - 10L dialysate |
| Urea | 33 | 237 |
| Creatinine | 8 | 12 |
| Phosphate | 7 | 12 |
| Potassium | 6 | 32 |

In one of the preferred treatment scenario's the nighttime cartridge is used overnight as a bedside device for 8 hours. After this treatment the peritoneal cavity is drained and filled with about 2L fresh dialysate filling, preferably based on icodextrin (such as Extraneal/Baxter). The filling stays over the day. Before starting a new session overnight, the peritoneal cavity is partly drained to reduce the intraperitoneal dialysate volume down to about 1L. This treatment is referred to extended cartridge + single dwell. For some patients it might be beneficial to further enhance the clearance during the day. This can be achieved by treatment with a single cartridge without extension (wearable) to allow mobility. The combined effect of the extended cartridge, daytime dwell and optionally the use of a single cartridge during daytime is listed in table 2.

**Table 2. Calculated clearance for an overnight treatment (8 hrs) with an extended cartridge (10L dialysate reservoir) in combination with a single dwell during daytime and in combination with a single dwell including treatment (8 hrs) with an additional daytime cartridge.**

| Clearance (ml/min) | Nighttime cartridge single dwell | Nighttime cartridge single dwell Daytime cartridge | Conventional PD |
|---|---|---|---|
| Urea | 9 | 10 | 6 |
| Creatinine | 10 | 15 | 4 |
| Phosphate | 6 | 9 | 4 |
| Potassium | 7 | 8 | 5 |

### Example 2: In vitro performance

Prototypes of the nighttime device with flow diagram as depicted in figures 6 were connected to a dialysate bags of 5L volume each, holding dialysate effluent collected from peritoneal dialysis patients (Fresenius Balance 1.5% filling). The prototypes were equipped with cartridges holding 325ml active carbon, 135ml phosphate binder (ferric oxyhydroxide) and were operated in tidal mode with a tidal volume of 300ml. The experiments were carried out with extended nighttime cartridges. The extension set comprised two electro-oxidation units (total electrode surface 2024cm2, operated at 2.9mA/cm2), 430ml potassium binder and 635ml active carbon. Total volume 1200ml. The dialysate bags were placed on a heating plate at 37C. The prototypes recirculated the effluent in tidal mode for 8 hours at a flow rate of 150ml/min. Every hour the effluent was spiked with urea, creatinine, phosphate and potassium to simulate transport of solutes from the intravascular space to the intraperitoneal cavity of a patient. The cumulative removal of toxins by the cartridges is listed in table 3.

**Table 3. In-vitro experiment, 5L PD effluent treated for 8 hrs. Toxin removal by a nighttime cartridge with a 1.2L extension set containing electro-oxidation, potassium binder and additional active carbon. In comparison the toxin removal of a nighttime cartridge with 10L dialysate reservoir extension (example 1).**

| Removal (mmol) | Nighttime cartridge/EO | Nighttime cartridge/Dialysate |
|---|---|---|
| | - 960ml carbon | - 325ml carbon |
| | - 135ml phosphate binder | - 135ml phosphate binder |
| | - 430ml potassium binder | - 10L dialysate (Physioneal 35) |
| | - 140ml electrodes | |
| Urea | 167 | 237 |
| Creatinine | 14.9 | 12 |
| Phosphate | 10.8 | 12 |
| Potassium | 36.3 | 32 |

### Example 3: In vivo performance

The efficacy of the prototype as described in example 1 was tested in vivo in a uremic pig model. The pig model was established by subtotal renal artery embolization and gentamicin administration. The performance was compared with that of a conventional static PD dwell (standard peritoneal permeability analysis, SPA, using Physioneal 35 1.36% glucose). Both the clearance and mass transfer area coefficient (MTAC) were determined. The results are given in table 4. A distinction is made between circumstances without peritonitis and with peritonitis (peritonitis is hard to avoid under the circumstance where the pigs are kept). Although the MTAC values of the pig model are quite low compared to human, the increase in MTAC thanks to the continuous tidal flow in the peritoneal cavity is quite evident. This effect will be more dominant at higher MTAC values as expected in humans. The relation between flow rate and MTAC is also very well visible in figure 12. Figure 12 represents the results of the in-vivo experiments with a large animal (pig) showing the increase in mass transfer coefficient (MTAC) when the flow rate of the dialysate is augmented. The results are obtained in a tidal mode operation.

**Table 4. In-vivo performance data of the device prototype**

| | Daytime cartridge | Nighttime cartridge 10L dialysate | Conventional PD SPA |
|---|---|---|---|
| *Clearance (ml*/*min)* | | | |
| No peritonitis | | | |
| Urea | | 5.8 | 5.8 |
| Creatinine | | 4.0 | 3.4 |
| Phosphate | | 3.5 | 2.7 |
| Peritonitis | | | |
| Urea | | 9.9 | 7.3 |
| Creatinine | 10.7 | 9.1 | 5.2 |
| Phosphate | 7.2 | 5.8 | 4.7 |

| *MTAC* (*ml*/*min*) | | | |
|---|---|---|---|
| No peritonitis | | | |
| Urea | | 11.3 | 8.7 |
| Creatinine | | 5.3 | 3.8 |
| Phosphate | | 4.3 | 2.8 |
| Peritonitis | | | |
| Urea | 18.2 | 20.7 | 16.8 |
| Creatinine | 16.8 | 13.8 | 8.9 |
| Phosphate | 11.1 | 8.6 | 7.1 |

## Claims

1. A device (1) for the removal of toxins from a patient by dialysis, **characterized in that** said device comprising:
i) a carrier (3) comprising the electronics (11) including control software, a user-interface (13), actuators (15) and sensors (17, 18, 19), which carrier (3) actuates, controls and monitors the dialysis operation;
ii) a replaceable cartridge (5) that is connected to the patient via a flexible tubing (7), which cartridge (5b) is connected to the carrier (3) and comprises:
• a reusable housing (21) with a pump (23), a leakage sensor (25), a memory chip (27) for cartridge identification and data storage,
• a purification unit (31) comprising a compartment (32) with tubings (33), connectors (34), check valves (35), a particle filter (36) and sorbents (37) and, in case of tidal mode operation, a tidal reservoir (39) for dialysate;
iii) an extension set (41) comprising a case (42) with a heating element (43), a temperature controller (44) and a power supply (45) for the heating element (43).

2. Device according to claim 1, **characterized in that** the sorbents (37) in the purification unit comprise:
i) active carbon for binding organic toxins and icodextrin or for buffering glucose hereby preventing that the peritoneal membrane is exposed to high glucose peaks, and
ii) an anion binder for removing phosphate and for buffering bicarbonate and/or lactate stabilizing the release of bicarbonate and/or lactate.

3. Device according to claim 1 or 2, **characterized in that** the particle filter (36) in the purification unit comprises a nanofilter with a pore size <10nm in order to protect the patient from possible fines released by the sorbents (35).

4. Device according to claim 3, **characterized in that** the nanofilter is a dialyzer filter (38), more specifically a low, medium or high flux filter with a typical poresize of 2-3nm.

5. Device according to any one of the preceding claims, **characterized in that** the extension set (41) comprises a dialysate reservoir (46) filled with fresh dialysate comprising NaCl, MgCl₂, CaCl₂, Na-bicarbonate and/or Na-lactate and glucose, icodextrin or other osmotic agent needed for ultrafiltration.

6. Device according to any one of the preceding claims 1-4, **characterized in that** the extension set (41) comprises a compartment with an electrocatalytic sorption unit for urea removal in combination with a potassium binding sorbent and a system for the release of glucose or similar osmotic agent in case of peritoneal dialysis and a suitable anti-oxidant.

7. Device according to any one of the preceding claims 1-5, **characterized in that** the extension set (41) comprises a compartment with sorbents for potassium and urea and fresh dialysate comprising NaCl, MgCl₂, CaCl₂, Na-bicarbonate and/or Na-lactate.

8. Device according to any one of the preceding claims, **characterized in that** the purification unit (31) can take the form of a disposable inlay.

9. Device according to claim 1, 2, 6 and 7, **characterized in that** the sorbents (37) in the purification unit (31) and the extension set (41) can be preloaded with electrolytes, bicarbonate, lactate, glucose or dextrin or other suitable osmotic agents for ultrafiltration and anti-oxidants such as ascorbic acid, N-acetyl cysteine, glutathione.

10. Device according to any one of the preceding claims, **characterized in that** the device is connected to the peritoneum of the patient via a single patient line to a single lumen or double lumen catheter (tidal mode) or via a double patient line to a double lumen catheter or two separate catheters (continuous mode).

11. Device according to claim 10, **characterized in that** the device comprises two separate catheters that can be operated both in continuous and tidal mode.

12. Device according to claim 10, **characterized in that** the device comprises a one port double lumen catheter (51) that is operated in tidal mode.

13. Device according to claim 10, **characterized in that** the device comprises a two port double lumen catheter (53) that can be operated both in continuous and tidal mode.

14. Device according to claim 10, **characterized in that** the device comprises a double patient line connected to a double lumen catheter or two separate catheters, a dialyzer filter (38) and two pump units (23) that can be operated in dialysis mode.

15. Device according to any one of the preceeding claims 1-2 and 4-9, **characterized in that** the device comprises a double patient line that can be connected directly to the blood circuit via a regular blood access such as a shunt, fistula or a central venous catheter, a dialyzer filter (38) and two pump units (23) that can be operated in dialysis mode.
